# FASCICULE DE BREVET EUROPEEN

(11) **EP 0 613 370 B1**
(45) Date de publication et mention de la délivrance du brevet: **28.06.1995**
(21) Numéro de dépôt: 93901758.8
(22) Date de dépôt: 23.11.1992
(51) Int. Cl.: A61K 9/54, A61K 9/24, A61K 31/55

(54) **FORMULATION A BASE DE DILTIAZEM A LIBERATION PROLONGEE**
ARZNEIMITTEL BESTEHEND AUS DILTIAZEM IN RETARDFORMEN
PROLONGED RELEASE DILTIAZEM-BASED FORMULATION

(30) Priorité: 22.11.1991 US 797496
(43) Date de publication de la demande: 07.09.1994
(73) Titulaire: LABORATOIRES PROGRAPHARM, 28170 Châteauneuf-en-Thymerais (FR)
(72) Inventeur: PHILIPPON, Francis, F-28500 Treon (FR); BOUTIN, Marie-Sylvie, F-28500 Saint-Gemme (FR); COUSSIN, Gérard, F-28320 Gallardon (FR); BRUNA, Etienne, F-28000 Chartres (FR)
(74) Mandataire: Koch, Gustave
(86) Numéro de dépôt international: FR9201083
(87) Numéro de publication internationale: WO9309767

(56) Documents cités:
- EP-A- 0 122 077
- US-A- 4 894 240
- US-A- 4 960 596
- US-A- 5 002 776

## Description

Le chlorhydrate de diltiazem-cis-(+)-3-(acétyloxy)-5-[2-(diméthylamino)-éthyl]-2,3-dihydro-2-(4-méthoxyphényl)-1,5-benzothiazépin-4(5H)-one est un dérivé du benzothiazine.

C'est un inhibiteur de l'influx de l'ion calcium, c'est-à-dire un agent bloquant les canaux lents ou antagoniste calcique. Il a un poids moléculaire de 450,98 et se présente sous la forme d'une poudre cristalline de couleur blanche à blanc cassé, soluble dans l'eau, dans le méthanol et le chloroforme.

Il a été démontré que le chlorhydrate de diltiazem soulage les symptômes des maladies cardiaques chroniques, en particulier de l'angine de poitrine et de l'ischémie myocardique, tout en ne présentant que de faibles effets secondaires.

Il est normalement administré par voie orale.

Le diltiazem est largement métabolisé par le foie et excrété par les reins et dans la bile. Bien que le mécanisme précis de son action anti-angineuse soit toujours en train d'être décrit, on pense que le diltiazem agit comme suit. Il a été montré que le diltiazem produit des augmentations du point de vue de la tolérance à l'effort probablement en raison de sa capacité à réduire la demande myocardique en oxygène. Ceci se traduit par une réduction du rythme cardiaque et de la pression sanguine systémique à des efforts submaximaux et maximaux. Chez l'animal, le diltiazem interfère avec le courant intérieur lent (dépolarisant) dans des tissus excitables. Il provoque le découplage excitation-contraction dans divers tissus myocardiques sans changer la configuration du potentiel d'action.

Le diltiazem produit la relaxation des fibres lisses vasculaires coronaires ainsi que la dilatation à la fois des grandes et des petites artères coronaires à des concentrations de substance active causant seulement un faible, voire aucun effet inotrope négatif. L'accroissement du flux sanguin coronaire (épicardique et subendocardique) qui en résulte dans les modèles ischémique et non-ischémique est accompagné par des décroissances de la pression sanguine systémique et de la résistance périphérique, dépendant de la dose administrée.

Comme d'autres antagonistes du calcium, le diltiazem diminue la conduction sinoauriculaire et atrioventriculaire dans des tissus isolés et produit un effet inotrope négatif sur les préparations isolées. Sur l'animal intact, on peut observer une prolongation de l'intervalle AH à des doses plus élevées.

Chez l'homme, le diltiazem empêche les spasmes coronaires artériels spontanés ou provoqués par l'ergonovine. Il provoque une diminution de la résistance vasculaire périphérique et une faible chute de la pression sanguine; dans des études de tolérance à l'effort conduites chez des patients présentant une maladie cardiaque ischémique, il réduit le double produit (débit x pression sanguine) au niveau du coeur pour toute charge de travail.

Formulé en comprimé, le diltiazem est absorbé à environ 80% par rapport à une capsule de référence et est soumis à un important effet de premier passage conduisant à une biodisponibilité absolue (comparée à l'administration intraveineuse) d'environ 40%.

Divers types de formulations de diltiazem à libération prolongée ont été décrits dans l'art antérieur ("The Use of Diltiazem Hydrochloride in Cardiovascular Disorders", McAuley and Schroeder, Pharmacotherapy, Vol. 2, No. 3, page 121, mai/juin 1982) et dans les brevets américains suivants: US-A-5 002 776, US-A-4 960 596, US-A-4 917 899, US-A-4 894 240, US-A-4 891 230 et US-A-4 721 619. Toutefois, tous ces brevets sauf l'un, à savoir le brevet US-A-4 960 596, impliquent l'utilisation d'un acide organique qui peut avoir un effet irritant.

L'invention a pour but, surtout, de fournir une formulation à effet prolongé à base de diltiazem qui ne présente plus les inconvénients des formulations de l'art antérieur, notamment pour une administration quotidienne en deux prises, qui ne présente pas l'effet irritant de ces formulations, dont la vitesse ou le profil de dissolution est satisfaisant.

Elle doit également
- pouvoir être fabriquée facilement avec un bon prix de revient,
- être adaptée pour une administration journalière en deux fois, ou en une fois,
- présenter une biodisponibilité relative équivalente à celle des formulations connues.

La formulation à base de diltiazem à libération prolongée pour administration orale conforme à l'invention comprend un microgranule comprenant tout d'abord une particule sphérique en sucre ou en sucre/amidon, une pluralité de couches primaires et secondaires alternées entourant la particule sphérique pour former un noyau, la couche alternative primaire comprenant un matériau polymère, soluble dans l'eau, pharmaceutiquement acceptable et la couche alternative secondaire, du diltiazem ou un de ses sels pharmaceutiquement acceptables, l'ensemble formant un noyau sur lequel sont appliquées deux membranes dont la première à partir du noyau est constituée par une couche d'un premier matériau polymère pharmaceutiquement acceptable et insoluble dans l'eau tel que de l'éthylcellulose additionnée de talc ou d'un autre agent de lubrification, et dont la deuxième à partir du noyau forme un revêtement en une seule couche relativement épaisse en un matériau polymère pharmaceutiquement acceptable et insoluble dans l'eau, tel que celui connu sous la marque EUDRAGIT S 100, additionné d'un agent plastifiant tel que le phtalate d'éthyle, de talc ou d'un autre agent lubrifiant.

Le nombre de couches d'enrobage de la membrane intérieure et de la membrane extérieure est tel que la libération du diltiazem à partir du noyau est effectuée à une vitesse permettant une absorption contrôlée de ce dernier en une période de huit, douze ou vingt heures après l'administration orale, cette vitesse qui est mesurée in vitro comme vitesse de dissolution du microgranule correspondant, lorsqu'elle est mesurée dans un milieu aqueux utilisant un appareil à palette tournante selon la Pharmacopée XXII des Etats-Unis, substantiellement au schéma de dissolution suivant: de 15 à 40% de la totalité du diltiazem sont libérés au bout de 4 heures de mesure; de 40 à 70% de la totalité du diltiazem sont libérés après 8 heures de mesure; de 50 à 85% de la totalité du diltiazem sont libérés après 12 heures de mesure et de 70 à 100% de la totalité du diltiazem sont libérés après 24 heures de mesure.

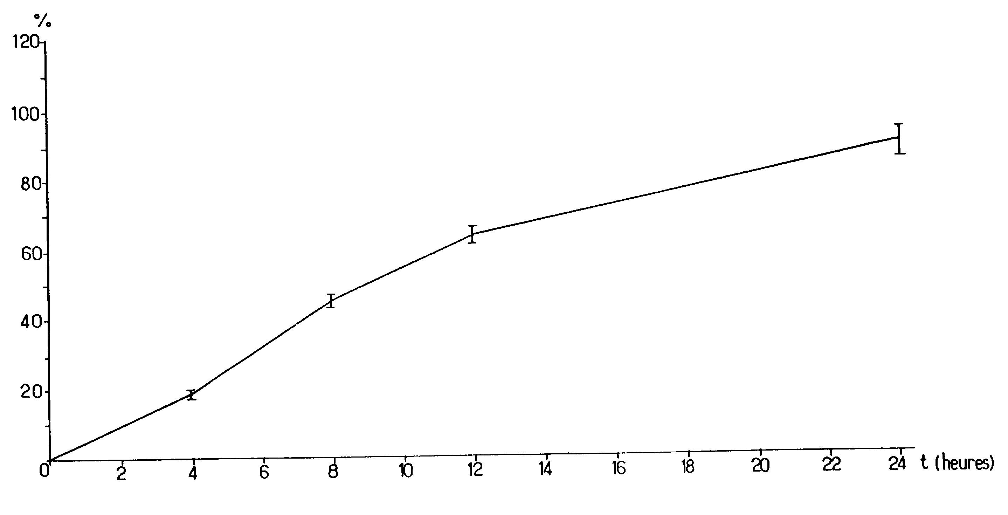

L'invention pourra être mieux comprise à l'aide de la description qui suit et à l'aide du dessin dans lequel la figure unique est une représentation graphique de la vitesse de dissolution du chlorhydrate de diltiazem à libération prolongée exprimée en pourcent (%) de substance active libérée en fonction du temps t (en heures) et tracée comme la moyenne des valeurs individuelles + Sx (écart type) obtenue en dissolvant la capsule échantillon de 120 mg dans 900 ml d'eau dans un appareil à palette tournant à une vitesse de 100 tours/minute conformément à la Pharmacopée XXII des Etats-Unis.

La formulation à base de diltiazem à libération prolongée conforme à la présente invention comprend des microgranules ou sphères "non pareils" ayant un diamètre moyen situé dans le domaine de 0,4 à 0,7 mm et comprenant des excipients neutres tels que le sucre ou le sucre/amidon. Des microgranules préparés conformément à l'invention contiennent de préférence environ 20 à 37% en poids de noyau neutre.

On applique sur le noyau une pluralité de couches alternées de diltiazem ou d'un sel pharmaceutiquement acceptable de ce dernier, tel que le chlorhydrate, et d'un matériau polymère soluble dans l'eau et pharmaceutiquement acceptable tel que la polyvinylpyrrolidone (PVP), telle que celle qui est connue sous la désignation PVP K 30, ledit diltiazem ou son sel étant appliqué par projection de la substance active sous forme de poudre.

D'autres polymères solubles dans l'eau et utilisables sont constitués par du polyéthylèneglycol ou de l'alcool polyvinylique ou par des mélanges de PVP et de l'un des produits qui précèdent.

Cet arrangement multicouche est obtenu dans une turbine conventionnelle utilisant des méthodes conventionnelles telles que la pulvérisation des ingrédients en vue de former la pluralité de couches successives d'ingrédient actif et de polymère.

Il convient de souligner qu'aucun autre produit tel qu'un acide organique ou tel que du talc n'est utilisé en association avec le chlorhydrate de diltiazem ou la PVP ou autre polymère.

On prépare une solution de PVP dans l'eau ou dans un solvant organique solubilisant (tel que l'alcool éthylique, l'acétone, l'isopropanol, l'eau ou des mélanges de ces produits) et le noyau central est humidifié à l'aide de la solution de PVP.

Une quantité appropriée de diltiazem est projetée ou appliquée par pulvérisation sous forme de poussière sur le noyau humidifié, après quoi on soumet ce dernier à une courte étape de séchage. L'application alternative de couches de PVP et de diltiazem est répétée jusqu'à ce que la quantité de diltiazem correspondant à la dose souhaitée soit obtenue.

La concentration du matériau polymère dans la solution est déterminée par la viscosité de la solution finale. Le rapport du noyau inerte au diltiazem est compris entre 1:1 et 1:10. Le noyau actif est obtenu par mise en oeuvre d'un procédé qui implique un certain nombre de cycles. Chacun de ces cycles ne conduit pas à une couche additionnelle à la surface du noyau mais plutôt à un élargissement ou à un grossissement de ce noyau.

De plus, à l'examen des microgranules finaux, il n'est pas possible de distinguer les couches dans le noyau actif alors que des couches effectives peut être vues distinctement dans la membrane. Dans un mode de réalisation avantageux, le nombre préféré de cycles est compris entre 70 et 140.

Le produit peut ensuite être séché dans de l'air chaud ou amené à passer à travers une enceinte de dessiccation. Les microgranules peuvent être soumis à un tamisage puis contrôlés du point de vue de leur humidité et de leur granulométrie.

De nombreux types de revêtements couverts par l'invention peuvent être utilisés pour fabriquer les membranes intérieures et extérieures et cela conformément à l'invention. Ces différents revêtements tels qu'envisagés par la présente invention permettent l'obtention de produits qui sont pour le moins biologiquement équivalents à des produits de l'art antérieur tels que ceux décrits dans le brevet U.S. N° 4.721.619.

La couche membranaire intérieure comprend de préférence de l'éthylcellulose et un lubrifiant tel que le talc. D'autres lubrifiants appropriés sont constitués par le stéarate de calcium, le stéarate de zinc ou de magnésium. Dans certains cas, la couche intérieure peut être constituée par un polymère tel que celui qui est connu sous la marque de fabrique EUDRAGIT RS.

Le revêtement membranaire extérieur comprend un matériau polymère insoluble dans l'eau et pharmaceutiquement acceptable qui est appliqué par dissolution du matériau dans un solvant approprié. De plus, la membrane peut contenir un agent plastifiant tel que le phtalate de diéthyle, et éventuellement un lubrifiant tel que le talc ou les autres lubrifiants identifiés ci-dessus.

Selon un mode de réalisation avantageux de l'invention, le matériau polymère insoluble dans l'eau constitutif de la membrane extérieure est de l'EUDRAGIT S 100. D'autres agents plastifiants appropriés sont constitués par les polyéthylèneglycols, le phtalate de dibutyle ou la triacétine (ou triacétate de glycérine). La membrane extérieure peut être constituée par une simple couche ou par jusqu'à cinq couches ou plus.

Le polymère commercialisé sous la marque EUDRAGIT S est faiblement perméable au diltiazem et à l'eau.

Les polymères vendus sous la marque EUDRAGIT sont constitués par des substances polymères de type vernis à base d'esters des acides acrylique et/ou méthacrylique. Ces produits sont décrits dans les brochures intitulées EUDRAGIT et émanant de la Société Röhm Pharma GmbH (1985 et suivantes). D'autres polymères appropriés insolubles dans l'eau sont constitués par ceux du groupe comprenant l'acétophtalate de cellulose, ceux commercialisés sous les marques de fabrique EUDRAGIT L, EUDRAGIT RS et AQUACOAT.

Dans le cadre de la présente invention, la solution d'éthylcellulose utilisée pour la membrane interne est préparée en utilisant un solvant. Ces ingrédients peuvent être mis en oeuvre par des méthodes conventionnelles, comme par exemple l'enrobage en turbine conventionel avec l'aide d'un agent lubrifiant versé dans la turbine. La solution peut être appliquée simultanément à la projection de talc. Le séchage peut être effectué dans l'air chaud, peu après quoi les microgranules sont soumis à un tamisage, l'humidité et la granulométrie étant ensuite contrôlées. De préférence, le nombre de couches de la membrane interne est compris entre 1 et 20.

Le revêtement membranaire extérieur est de préférence appliqué par une méthode conventionnelle mettant en oeuvre un lit fluidisé équipé d'une colonne de Würster dans le cadre d'un procédé en continu. Dans cet équipement, la membrane extérieure appropriée est pulvérisée sur les noyaux revêtus d'éthylcellulose. La solution à partir de laquelle est fabriquée la membrane extérieure peut contenir un ou plusieurs polymères insolubles dans l'eau, dissous dans un solvant approprié tel que l'éthanol, l'acétone ou l'isopropanol ou dans un mélange de ces produits en présence d'autres additifs tels qu'un agent de lubrification, par exemple le talc, et un agent plastifiant tel que le phtalate de diéthyle. Le revêtement obtenu est très homogène et régulier. Le revêtement membranaire extérieur peut comprendre de une à cinq couches ou plus. Le séchage peut être effectué dans l'air chaud, après quoi les microgranules sont soumis à un tamisage avant contrôle de l'humidité et de la granulométrie. Ensuite, le produit peut être introduit dans des capsules ou dans des comprimés.

A aucun moment, on n'utilise de gomme laque dans l'une ou l'autre des membranes. La gomme laque n'est pas satisfaisante pour différentes raisons, y compris la diminution de la durée de stockage du produit.

Les caractéristiques des membranes insolubles dans l'eau, intérieures et extérieures, du produit ainsi obtenu sont sensées rendre possible la libération du chlorhydrate de diltiazem dans un milieu aqueux suivant les intervalles indiqués ci-après et mesurés en utilisant un appareil à palette tournante selon la Pharmacopée XXII des Etats-Unis d'Amérique:
(a) de 15 à 40% de la totalité du diltiazem sont libérés après 4 heures de mesure,
(b) de 40 à 70% de la totalité du diltiazem sont libérés après 8 heures de mesure,
(c) de 50 à 85% de la totalité du diltiazem sont libérés après 12 heures de mesure, et
(d) de 70 à 100% de la totalité du diltiazem sont libérés après 24 heures de mesure.

Il résulte de ce qui précède que la membrane préférée conforme à la présente invention contient une combinaison de deux ou de plusieurs polymères insolubles dans l'eau dont l'un est soluble dans les milieux neutres ou faiblement alcalins. L'un de ces polymères, l'éthylcellulose, est insoluble dans l'eau et insoluble dans des milieux neutres ou faiblement alcalins. Un autre de ces polymères, à savoir celui qui est commercialisé sous la marque EUDRAGIT S 100, est insoluble dans l'eau mais soluble dans des milieux neutres ou faiblement alcalins.

Dans les modes de réalisation appropriés, il y a approximativement 10% de copolymère de l'acide méthacrylique, 0,6% d'éthylcellulose N7, 1,25% d'agent plastifiant et 6,5% de talc. Ces pourcentages aussi bien que tous autres pourcentages indiqués ci-dessus sont des pourcentages en poids.

Le rapport en poids du polymère insoluble dans l'eau et dépendant du pH, tel que celui commercialisé sous la marque EUDRAGIT S 100, par rapport au diltiazem se situe dans le domaine de 0,15:1 à 0,24:1.

Le rapport du polymère insoluble dans l'eau et indépendant du pH, tel que l'éthylcellulose, se situe dans le domaine de rapport en poids allant de 0,009:1 à 0,014:1.

Le rapport de l'agent lubrifiant au diltiazem se situe dans le domaine de rapport en poids allant de 0,1:1 à 0,15:1.

Le rapport de l'agent plastifiant au diltiazem se situe dans le domaine de rapport en poids allant de 0,02:1 à 0,03:1.

### EXEMPLE DE PREPARATION I

On fait passer par tamisage à travers une grille de 1,25 mm, une quantité de 6,0 kg de chlorhydrate de diltiazem. La poudre est appliquée sur des microgranules en amidon/sucre (diamètre de 0,4 mm à 0,7 mm) dont une quantité de 3,6 kg est disposée dans une turbine, en utilisant une solution liante à 20% de PVP K 30 dans l'alcool.

Les microgranules sont enrobés avec un volume mesuré de solution liante suivi par l'application par saupoudrage d'une quantité mesurée de poudre. Les microgranules enrobés sont laissés à sécher et cette étape de formation de couches est répétée jusqu'à ce que toute la poudre ait été appliquée.

Les microgranules enrobés sont ensuite séchés pendant la nuit à environ 25°C.

Les microgranules sont ensuite soumis à un test de contrôle avant application du revêtement.

Les noyaux actifs, une fois préparés, sont entourés d'une première membrane ou membrane intérieure en appliquant 12 couches d'une solution constituée de 10% du produit vendu sous la marque EUDRAGIT RS dans une solution acétone/isopropanol à l'intérieur d'une turbine. Du talc est appliqué par saupoudrage à l'intérieur de la turbine pendant cette étape. Le produit commercialisé sous la marque EUDRAGIT RS est un polymère anionique synthétisé à partir d'ester des acides acrylique et méthacrylique et comportant une faible teneur de groupe ammonium quaternaire. Les groupes ammonium sont présents en tant que sels et donnent lieu à la perméabilité de la pellicule de vernis. Ils fournissent des revêtements sous forme de pellicules insolubles dans l'eau mais perméables.

Les microgranules ainsi obtenus sont ensuite introduits dans un lit fluidisé équipé d'une colonne de Würster. Dans cet équipement, une solution polymère mélangée avec du phtalate de diéthyle et du talc constituant une membrane extérieure ou secondaire, est appliquée par pulvérisation sur les microgranules. La solution polymère est constituée de 70% (poids/poids) d'une solution à 7,5% d'EUDRAGIT S dans de l'acétone/isopropanol et de 30% (poids/poids) d'une solution à 7,5% d'EUDRAGIT L dans l'acétone/isopropanol.

### EXEMPLE DE PREPARATION II

L'exemple I est repris, à la différence près que la solution polymère utilisée pour la seconde membrane est constituée à raison de 25% (poids/poids) d'une solution à 7,5% d'EUDRAGIT L dans l'acétone/isopropanol et à raison de 75% (poids/poids) d'une solution à 7,5% d'EUDRAGIT S dans l'acétone/isopropanol.

### EXEMPLE DE PREPARATION III (mode de réalisation préféré)

L'exemple I est répété, à la différence près que la solution polymère utilisée pour la première membrane a été de l'éthylcellulose à la place du produit commercialisé sous la marque EUDRAGIT RS et la solution polymère utilisée pour la membrane extérieure est constituée seulement du produit commercialisé sous la marque EUDRAGIT S.

### Résultats réunis en rapport avec la dissolution

Le produit selon l'exemple III a été utilisé dans des essais cliniques effectués sur l'homme.

Les résultats obtenus, qui sont relatifs au produit industriel, sont comme suit:

| pH = 6,5 (USP XXII) | | | |
|---|---|---|---|
| Temps (h) | Moyenne (6 essais) % | Déviation standard | cv % |
| 1 | 2,14 | 0,39 | 18,42 |
| 2 | 6,06 | 0,53 | 8,79 |
| 3 | 11,65 | 0,69 | 5,89 |
| 4 | 18,18 | 0,74 | 4,06 |
| 5 | 25,15 | 0,90 | 3,57 |
| 6 | 32,24 | 0,95 | 2,94 |
| 7 | 39,39 | 0,80 | 2,02 |
| 8 | 45,66 | 0,94 | 2,06 |

| pH = 7,4 (USP XXII) | | | |
|---|---|---|---|
| Temps (h) | Moyenne (6 essais) % | Déviation standard | cv % |
| 1 | 31,00 | 3,82 | 12,32 |
| 2 | 87,13 | 2,22 | 2,54 |
| 3 | 98,43 | 1,40 | 1,43 |
| 4 | 101,88 | 1,17 | 1,15 |

| pH = 8,5 (USP XXII) | | | |
|---|---|---|---|
| Temps (h) | Moyenne (6 essais) % | Déviation standard | cv % |
| 1 | 99,66 | 2,06 | 2,06 |
| 2 | 98,81 | 1,16 | 1,17 |
| 3 | 94,45 | 0,84 | 0,89 |

| Eau | | | |
|---|---|---|---|
| Temps (h) | Moyenne (6 essais) % | Déviation standard | cv % |
| 4 | 16,68 | 1,77 | 10,58 |
| 8 | 43,96 | 0,96 | 2,19 |
| 12 | 62,47 | 0,78 | 1,25 |
| 24 | 86,46 | 1,95 | 2,26 |

### Résultats du test de dissolution / Premier exemple de fabrication

| Eau | |
|---|---|
| Temps (h) | Moyenne (3 essais) % |
| 4 | 32,83 |
| 8 | 62,66 |
| 12 | 77,81 |
| 24 | 94,92 |

### Résultats du test de dissolution / Second exemple de fabrication

| Eau | |
|---|---|
| Temps (h) | Moyenne (3 essais) % |
| 4 | 37,71 |
| 8 | 66,89 |
| 12 | 80,15 |
| 24 | 95,60 |

### Résultats du test de dissolution / Troisième exemple de fabrication (concernant un lot de laboratoire)

| Eau | | | |
|---|---|---|---|
| Temps (h) | Moyenne (6 essais) % | Déviation standard | cv % |
| 4 | 26,09 | 1,76 | 6,76 |
| 8 | 43,96 | 1,28 | 2,91 |
| 12 | 57,16 | 1,08 | 1,90 |
| 24 | 78,43 | 0,99 | 1,26 |

### EXEMPLES DE CAPSULES

On indique ci-après un exemple de capsule à libération prolongée contenant 60 mg de chlorhydrate de diltiazem:

| | |
|---|---|
| Chlorhydrate de diltiazem, USP | 60 mg |
| Sphères de sucre, NF | 33,3 mg |
| Povidone, USP | 3,9 mg |
| Copolymère d'acide méthacrylique, NF | 11,9 mg |
| Ethylcellulose, NF | 0,7 mg |
| Phtalate de diéthyle, NF | 1,5 mg |
| Talc, USP | 7,5 mg |
| Alcool isopropylique, USP | non résiduel |
| Acétone | non résiduel |
| Alcool | non résiduel |
| TOTAL | 118,8 mg |

Capsule en gélatine #3, les colorants étant constitués par de l'oxyde de fer rouge et du dioxyde de titane.

On indique ci-après un exemple de capsule à libération prolongée contenant 90 mg de chlorhydrate de diltiazem:

| | |
|---|---|
| Chlorhydrate de diltiazem, USP | 90 mg |
| Sphères de sucre, NF | 50 mg |
| Povidone, USP | 5,9 mg |
| Copolymère d'acide méthacrylique, NF | 17,8 mg |
| Ethylcellulose, NF | 1 mg |
| Phtalate de diéthyle, NF | 2,2 mg |
| Talc, USP | 11,2 mg |
| Alcool isopropylique, USP | non résiduel |
| Acétone | non résiduel |
| Alcool | non résiduel |
| TOTAL | 178,1 mg |

Capsule en gélatine #2, les colorants étant constitués par de l'oxyde de fer rouge, par de l'oxyde de fer jaune et du dioxyde de titane.

On indique ci-après un exemple de capsule à libération prolongée contenant 120 mg de chlorhydrate de diltiazem:

| | |
|---|---|
| Chlorhydrate de diltiazem, USP | 120 mg |
| Sphères de sucre, NF | 66,6 mg |
| Povidone, USP | 7,8 mg |
| Copolymère d'acide méthacrylique, NF | 23,8 mg |
| Ethylcellulose, NF | 1,4 mg |
| Phtalate de diéthyle, NF | 3 mg |
| Talc, USP | 15 mg |
| Alcool isopropylique, USP | non résiduel |
| Acétone | non résiduel |
| Alcool | non résiduel |
| TOTAL | 237,6 mg |

Capsule en gélatine #1, les colorants étant constitués par de l'oxyde de fer rouge, de l'oxyde de fer jaune et du dioxyde de titane.

Dans les trois exemples de capsules susdits il apparaît clairement à l'homme de l'art que la totalité de la quantité d'ingrédients actifs par capsule variera d'un lot à l'autre, en rapport avec la quantité des microgranules appropriée pour la libération du nombre précis de milligrammes de chlorhydrate de diltiazem par capsule. La quantité d'excipients peut varier jusqu'à ±20%.

La quantité de microgranules par capsule peut être déterminée avant le remplissage des capsules à l'aide du résultat de la teneur en chlorhydrate de diltiazem du lot de microgranules de chlorhydrate de diltiazem.

## Revendications

1. Formulation à base de diltiazem à libération prolongée pour administration orale conprenant un microgranule exempt d'acide organique pouvant avoir un effet irritant, ledit microgranule comprenant:
(a) une particule sphérique inactive centrale;
(b) une pluralité de couches primaires et secondaires en alternance entourant la particule sphérique pour former un noyau, la couche primaire comprenant un matériau polymère pharmaceutiquement acceptable, soluble dans l'eau, et la couche secondaire comprenant le diltiazem ou un de ses sels pharmaceutiquement acceptables; et
(c) un revêtement extérieur comprenant
des premières couches membranaires intérieures appliquées audit noyau, lesdites premières couches membranaires intérieures qui sont au nombre de 1 à 20 comprenant un premier matériau polymère pharmaceutiquement acceptable, insoluble dans l'eau, et
une seule membrane extérieure formant une couche relativement épaisse et homogène entourant lesdites premières couches membranaires intérieures et comprenant un second matériau polymère pharmaceutiquement acceptable, insoluble dans l'eau, différent du premier matériau polymère pharmaceutiquement acceptable, insoluble dans l'eau.

2. Formulation selon la revendication 1, dans laquelle le matériau polymère pharmaceutiquement acceptable, soluble dans l'eau, est choisi dans le groupe comprenant la polyvinylpyrrolidone, le polyéthylèneglycol, l'alcool polyvinylique et leurs mélanges. .

3. Formulation selon la revendication 1, dans laquelle le premier matériau polymère pharmaceutiquement acceptable, insoluble dans l'eau, est l'éthylcellulose et le second matériau polymère pharmaceutiquement acceptable, insoluble dans l'eau, est choisi dans le groupe comprenant un copolymère d'esters d'acide acrylique et/ou méthacrylique, l'acétatophtalate de cellulose, et ceux commercialisés sous les marques EUDRAGIT L, EUDRAGIT RS et AQUACOAT.

4. Formulation selon la revendication 1, comprenant un lubrifiant dans les couches membranaires primaires et dans la membrane extérieure simple, ledit lubrifiant étant choisi dans le groupe comprenant le talc, le stéarate de calcium, le stéarate de zinc ou le stéarate de magnésium.

5. Formulation selon la revendication 1, comprenant un agent plastifiant dans la seule membrane extérieure.

6. Formulation selon la revendication 5, dans laquelle ledit agent plastifiant est choisi dans le groupe comprenant le phtalate de diéthyle, les polyéthylèneglycols, le phtalate de dibutyle et la triacétine.

## Claims

1. Sustained release diltiazem formulation for oral administration comprising a microgranule free of organic acid possibly having an irritating effect, the said microgranule comprising:
(a) a central inactive spherical particle;
(b) a plurality of alternating first and second layers surrounding the spherical particle to form a core, the first layer comprising a water soluble, pharmaceutically acceptable polymeric material, and the second layer comprising diltiazem or a pharmaceutically acceptable salt thereof; and
(c) an outer coating comprising
first inner membrane layers applied to said core, said first inner membrane layers, amounting from 1 to 20, comprising a first water insoluble pharmaceutically acceptable polymeric material, and
a single outer membrane forming a relatively thick and homogeneous layer surrounding said first inner membrane layers and comprising a second water insoluble pharmaceutically acceptable polymeric material different from said first water insoluble pharmaceutically acceptable polymeric material.

2. Formulation according to claim 1, wherein the water soluble, pharmaceutically acceptable polymeric material is selected from the group comprising polyvinylpyrrolidone, polyethyleneglycol, polyvinyl alcohol and their mixtures.

3. Formulation according to claim 1, wherein the first water insoluble, pharmaceutically acceptable polymeric material is ethylcellulose and wherein the second water insoluble, pharmaceutically acceptable polymeric material is selected from the group comprising a copolymer of acrylic and/or methacrylic acid esters, cellulose acetate phthalate, and those marketed under the trademarks EUDRAGIT L, EUDRAGIT RS and AQUACOAT.

4. Formulation according to claim 1, including a lubricant in said first membrane layers and in said single outer membrane, said lubricant being selected from the group comprising talc, calcium stearate, zinc stearate or magnesium stearate.

5. Formulation according to claim 1, comprising a plasticizer in said single outer membrane.

6. Formulation according to claim 5, wherein said plasticizer is selected from the group comprising diethylphthalate, polyethyleneglycols, dibutyl phthalate and triacetin.

## Patentansprüche

1. Formulierung auf Basis von Diltiazem mit retardierter Freisetzung zur oralen Verabreichung, umfassend ein Mikrokörnchen welches frei ist von organischer Säure die eine Reizwirkung hervorrufen kann, wobei das Mikrokörnchen umfaßt:
(a) ein rundliches inaktives zentrales Teilchen,
(b) mehrere erste und zweite Schichten, die abwechselnd das rundliche Teilchen umgeben um einen Kern zu bilden, wobei die erste Schicht ein pharmazeutisch verträgliches, wasserlösliches Polymermaterial umfaßt und die zweite Schicht das Diltiazem oder eines seiner pharmazeutisch verträglichen Salze umfaßt, und
(c) eine äußere Umhüllung, umfassend
erste innere membranartige Schichten, welche auf dem Kern aufgebracht sind, wobei die inneren membranartigen Schichten, die in einer Zahl von 1 bis 20 vorhanden sind, ein erstes pharmazeutisch verträgliches, wasserunlösliches Polymermaterial umfassen, und
eine einzige äußere Membran, die eine relativ dicke und homogene, die inneren Membranen umgebende Schicht bildet, und ein zweites pharmazeutisch verträgliches, wasserunlösliches Polymermaterial, das vom ersten pharmazeutisch verträglichen, wasserunlöslichen Polymermaterial unterschiedlich ist, umfaßt.

2. Formulierung nach Anspruch 1, wobei das erste pharmazeutisch verträgliche, wasserlösliche Polymermaterial ausgewählt ist aus der Gruppe umfassend Polyvinylpyrrolidon, Polyethylenglykol, Polyvinylalkohol und Gemische davon.

3. Formulierung nach Anspruch 1, wobei das erste pharmazeutisch verträgliche, wasserunlösliche Polymermaterial Ethylcellulose ist und das zweite pharmazeutisch verträgliche, wasserunlösliche Polymermaterial ausgewählt ist aus der Gruppe umfassend ein Copolymer von Acryl- und/oder Methacrylsäureestern, Celluloseacetatphthalat und die unter den Markenbezeichnungen EUDRAGIT L, EUDRAGIT RS und AQUACOAT vertriebenen.

4. Formulierung nach Anspruch 1, umfassend ein Gleitmittel in den ersten membranartigen Schichten und in der einzigen äußeren Membran, wobei das Gleitmittel ausgewählt wird aus der Gruppe umfassend Talk, Calciumstearat, Zinkstearat und Magnesiumstearat.

5. Formulierung nach Anspruch 1, umfassend ein Plastifizierungsmittel in der einzigen äußeren Membran.

6. Formulierung nach Anspruch 5, wobei das Plastifizierungsmittel ausgewählt ist aus der Gruppe umfassend Diethylphthalat, Polyethylenglykole, Dibutylphthalat und Triacetin.
